# EUROPEAN PATENT APPLICATION

(11) **EP 4 606 811 A1**
(43) Date of publication of application: **27.08.2025**
(21) Application number: 22962365.7
(22) Date of filing: 19.10.2022
(51) Int. Cl.: C07H 5/02, C07H 1/06

(54) **PREPARATION METHOD FOR SUCRALOSE REFINED PRODUCT**

(71) Applicant: Anhui Jinhe Industrial Co., Ltd., Chuzhou, Anhui 239200 (CN)
(72) Inventor: CHEN, Yongle, Chuzhou, Anhui 239200 (CN); SHEN, Dongdong, Chuzhou, Anhui 239200 (CN); CHEN, Yuhan, Chuzhou, Anhui 239200 (CN); XIAO, Shidong, Chuzhou, Anhui 239200 (CN)
(74) Representative: Ipside
(86) International application number: PCT/CN2022/126183
(87) International publication number: WO 2024/082175

(57) **Abstract**

The present invention provides a preparation method for a sucralose refined product, and relates to the technical field of fine chemical engineering. In the present invention, by means of subjecting a sucralose crude product to ethyl acetate pulping treatment, two instances of activated carbon decolorization and impurity removal at different stages and three instances of crystallization, impurities in the sucralose crude product and impurities generated by oxidation during a crystallized mother liquor use process can be effectively removed, and the problem of impurity enrichment during the recycling process of a crystallized mother liquor is solved, such that crystallized mother liquor generated during the refining process can be recycled, the loss of sucralose during the purification process is reduced, and the yield of sucralose is significantly improved. Furthermore, the preparation method for a sucralose refined product provided in the present invention can be effectively combined with a preparation method for a sucralose crude product, such that the mother liquor formed during the preparation process of the sucralose refined product can be recycled, the yield of sucralose is significantly increased, the production cost is significantly reduced, and the preparation method has relatively good industrial prospects.

## Description

### Technical Field

The present invention relates to the technical field of fine chemical engineering, in particular to a preparation method for a sucralose refined product.

### Background of the Invention

Sucralose, also known as Splenda, is white crystalline powder or granules in appearance, is a new kind of sweetener using sucrose as a raw material, almost 600 times as sweet as sucrose, has pure taste, does not participate in human metabolism, can be used as 'zero calorie' sugar for patients with diabetes, cardiovascular and cerebrovascular diseases and the elderly, has the characteristics of good stability, high safety,etc., and is widely applied to a plurality of fields of foods, beverages, daily chemicals, medicines, etc.
At present, the production process of sucralose mainly uses sucrose as a raw material to obtain sucrose-6-acetate through esterification, sucrose-6-acetate is chlorinated to obtain sucralose-6-acetate, and then sucralose-6-acetate is alcoholyzed and purified to obtain refined sucralose. Among them, the synthesis of sucrose-6-acetate mainly includes trimethyl orthoacetate process (e.g., Chinese patents CN106749440A, CN105254684A) and organotin process (for example, Chinese patents CN102639550A, CN 1528772A). The synthesis of sucralose-6-acetate is mainly phosgene/solid phosgene process (CN103328495A, CN1660868A) and thionyl chloride process (for example, Chinese patent CN101270136A, CN 102417526A). There are many methods for producing sucralose from sucralose-6-acetate, such as sodium methoxide method (e.g., Chinese patents CN101918421A, CN1176094A), organic amine method (e.g., Chinese patents CN101260127A, CN112771060A, CN101260127A), alkaline ion exchange resin method (e.g., Chinese patents CN112409419A, CN102336787A), alkali metal oxide method (e.g., Chinese patents CN113004345A, CN104004032A, CN112805291A), and alkali metal hydroxide method (e.g., Chinese patents CN1814609A, CN101012250A, CN 102321122A).

The traditional production method of sucralose refined product mainly comprises the following steps: taking sucralose-6-acetate pure product (the purity is more than 99%) as a raw material, preparing sucralose crude product through alcoholysis (sodium methoxide/methanol) or alkaline hydrolysis (liquid caustic soda/water), purifying (extracting by butyl acetate to remove impurities, then crystallizing in water) to obtain sucralose refined product, wherein in the purification process, the crystallized mother liquor is recycled, and needs to be concentrated in the recycling process. However, the above purification steps may cause sucralose to be oxidized to form new impurities, the new impurities are enriched in the cyclic utilization process of the crystallized mother liquor, and further affect the crystallization of sucralose, and in this case, the crystallized mother liquor (which has been recycled about five times) can no longer be recycled and has to be treated as wastewater instead.

### Summary of the Invention

In view of this, the present invention provides a preparation method of a sucralose refined product, in which the crystallized mother liquor can be recycled many times, and the sucralose refined product has high yield and high purity.

In order to achieve the above object, the present invention provides the following technical solutions:
The invention provides a preparation method of a sucralose refined product, which comprises the following steps:
The invention provides a preparation method of a sucralose refined product, which comprises the following steps:
(1) Pulping and decoloring: mixing the sucralose crude product with ethyl acetate, pulping, and then carrying out solid-liquid separation to obtain a pulped mother liquor and a pulped target product respectively; dissolving the pulped target product in water, and decoloring the obtained aqueous solution of the pulped target product by using activated carbon to obtain a pulped and decolored target product solution;
(2) First crystallization: concentrating the pulped and decolored target product solution, and performing first crystallization on the obtained pulped and decolored target product concentrated solution to obtain a first crystallized target product and a primary crystallized mother liquor respectively;
(3) Second crystallization: dissolving the first crystallized target product in water, and carrying out second crystallization on the obtained first crystallized target product aqueous solution to obtain a second crystallized target product and a secondary crystallized mother liquor respectively; the secondary crystallized mother liquor is recycled to step (1) for dissolving the pulped target product;
(4) Third crystallization: dissolving the second crystallized target product in water, and performing solid-liquid separation on the obtained second crystallized target product aqueous solution after activated carbon decolorization to obtain a second crystallized decolorized target product aqueous solution and recovered activated carbon respectively; concentrating the second crystallized decolorized target product aqueous solution, and carrying out third crystallization on the obtained second crystallized decolorized target product concentrated solution to obtain a third crystallized target product and a tertiary crystallized mother liquor respectively; drying the tertiary crystallized target product to obtain a sucralose refined product; recycling the tertiary crystallized mother liquor to step (3) for dissolving the first crystallized target product, and recycling the recovered activated carbon to step (1) for decoloring the pulped target product aqueous solution.

Preferably, in step (1), the sucralose crude product compromises the following components in percentage by mass: 85-95% of sucralose, 0.5-1% of sucralose-6-acetate, 1-3% of ethyl acetate and 1-13.5% of caramel impurities.

Preferably, in step (1), the ratio of the mass of the sucralose crude product to the volume of the ethyl acetate for pulping is 1 kg: 1-1.5 L.

Preferably, in step (1), the dissolving temperature is 30-50 °C;
The sugar degree of the pulped target product aqueous solution is 20-40%.

Preferably, in step (2), the sugar degree of the pulped and decolored target product concentrated solution is 40-65%.

Preferably, in step (2), the temperature of the first crystallization is room temperature, and the time is 8-16 h.

Preferably, in step (3), the dissolving temperature is 40-60 °C;
The sugar degree of the first crystallized target product aqueous solution is 40-65%.

Preferably, in step (3), the temperature of the second crystallization is room temperature, and the time is 8-16 h.

Preferably, in step (4), the dissolving temperature is 40-60 °C;
The sugar degree of the second crystallized decolorized target product aqueous solution is 20-30%.

Preferably, in step (4), the sugar degree of the second crystallized decolorized target product concentrated solution is 40-65%.

Preferably, in step (4), the temperature of the third crystallization is 30-50 °C, and the time is 4-12 h.

Preferably, the preparation method of the sucralose crude product comprises the following steps:
(a) Extracting the raw material liquid by ethyl acetate to obtain a first ethyl ester phase and a first aqueous phase respectively; concentrating the first ethyl ester phase to obtain a first ethyl ester phase concentrate; the raw material liquid is an aqueous solution containing sucralose-6-acetate, sucralose diester and sucralose tetrachloride-6-acetate;
(b) Mixing and dissolving the first ethyl ester phase concentrate and water, and concentrating to obtain a sucralose-6-acetate aqueous solution;
(c) Mixing the sucralose-6-acetate aqueous solution with an alkali metal hydroxide for carrying out an alkaline hydrolysis reaction, neutralizing the obtained reaction solution, and filtering to obtain a second aqueous phase;
(d) Performing ethyl acetate extraction on the second aqueous phase to obtain a second ethyl ester phase and a third aqueous phase respectively;
(e) Washing the second ethyl ester phase with water to obtain a fourth aqueous phase and a third diethyl ester phase respectively; said fourth aqueous phase is recycled to step (b) for dissolving said first ethyl ester phase concentrate;
(f) Mixing the third ethyl ester phase with ethyl acetate, and carrying out azeotropic dehydration to obtain a third ethyl ester phase concentrate; mixing the third ethyl ester phase concentrate with ethyl acetate to obtain a fourth ethyl ester phase;
(g) Crystallizing the fourth ester phase to obtain a sucralose crude product and a fifth ethyl ester phase respectively;
(h) Washing the fifth ethyl ester phase with water to obtain a fifth aqueous phase and a sixth ethyl ester phase respectively; the fifth aqueous phase is recycled to step (b) for dissolving the first ethyl ester phase concentrate.

Preferably, the pulped mother liquor and the primary crystallized mother liquor are recycled for preparing sucralose crude product.

Preferably, During the recycling, the pulped mother liquor is used for dissolving the first ethyl ester phase concentrate, and after primary crystallized mother liquor and the third ethyl ester phase are mixed, azeotropic dehydration is carried out.

Preferably, the sucralose content in the third, fourth, and fifth aqueous phases is independently < 0.5 g/L.

Preferably, in step (a), the temperature of ethyl acetate extraction is 40-60 °C, and the number of extraction times is 5-8; the volume ratio of the raw material liquid to ethyl acetate for single ethyl acetate extraction is 1: 0.2-0.5.

Preferably, in step (b), the content of ethyl acetate in the sucralose-6-acetate aqueous solution is < 0.5 g/L.

Preferably, in step (c), the pH value of the alkaline hydrolysis reaction is 11-13, the temperature is 0-10 °C, and the time is 3-6 h.

Preferably, in step (c), the temperature of the neutralization and the filtration is 50-70 °C independently.

Preferably, in step (d), the number of times of ethyl acetate extraction is 4-7; the volume ratio of the second aqueous phase to the ethyl acetate used for single-time extraction is 1: 1-3;
The ethyl ester phases obtained by the first to the second ethyl acetate extraction are combined as a second ethyl ester phase;
The ethyl acetate phases obtained by the third to the seventh ethyl acetate extraction are used for the ethyl acetate extraction of the second aqueous phase in the next batch of sucralose crude product preparation process.

Preferably, in step (e), the number of times of water washing is 3-6; the volume ratio of the second ethyl ester phase to the water used for single-time water washing is 1: 0.1-0.3;
The aqueous phase obtained by the first water washing is taken as a fourth aqueous phase;
The aqueous phases obtained by the second to the sixth water washing are used for the water washing of the second ethyl ester phase in the next batch of sucralose crude product preparation process.

Preferably, in step (f), the water content of the third ethyl ester phase concentrate is < 0.5 wt%.

Preferably, in step (f), the sugar degree of the fourth ethyl ester phase is 40-60%.

Preferably, in step (g), the crystallization temperature is 40-60 °C, and the time is 12-30 h.

The prior art can not realize the material balance problem between the target product and the impurities in the process of using the sucralose crude product as the raw material to obtain the sucralose refined product, and finally, after the mother liquor is recycled for a plurality of times, the mother liquor has to be discarded because of excessive impurities. According to the preparation method provided in the invention, the sucralose crude product is subjected to ethyl acetate pulping treatment, two times of activated carbon decolorization and impurity removal at different stages and three times of crystallization, so that impurities in the sucralose crude product and impurities generated by oxidation in the process of recycling the crystallized mother liquor can be effectively removed, the stability of the subsequent crystallized mother liquor is ensured, and the problem of impurity enrichment in the process of recycling the crystallized mother liquor is solved, so that the crystallized mother liquor generated during the refining process can be recycled repeatedly, the loss of sucralose in the purification process is reduced, and the yield of sucralose is significantly improved. According to the invention, the secondary crystallized mother liquor is reused for dissolving the pulped target product, and the tertiary crystallized mother liquor is reused for dissolving the first crystallized target product, so that residual sucralose in the secondary crystallized mother liquor and the tertiary crystallized mother liquor can be fully utilized, a large amount of loss caused by sucralose in the crystallization process is reduced, and the yield of sucralose is obviously improved.

Furthermore, the pulped mother liquor contains a large amount of fat-soluble impurities and part of sucralose, and the primary crystallized mother liquor contains residual sucralose. In the present invention, by using the pulped mother liquor and the primary crystallized mother liquor to prepare the sucralose crude product, the loss of sucralose can be avoided and the overall yield of sucralose can be improved. The preparation method provided in the invention can effectively realize the effective combination of the preparation of the sucralose crude product and the purification of the sucralose crude product, realize the balance of sucralose and impurities in the purification process, and realize the long-term repeated cyclic utilization of the mother liquor.

Furthermore, the invention carries out alkaline hydrolysis in an alkali metal hydroxide-water system, sucralose-6-acetate, sucralose diester and sucralose-6-acetate tetrachloride will undergo alkaline hydrolysis in the presence of a strong alkaline aqueous solution to generate the corresponding sucralose and sucralose tetrachloride, the sucralose tetrachloride can be continuously dechlorinated in the presence of a strong alkaline aqueous solution to form sucralose, so that the sucralose-6-acetate and impurities (sucralose diester and sucralose tetrachloride-6-acetate) in the raw material liquid are converted into sucralose, therefore, the conversion rate of the raw materials and the yield of the sucralose are obviously improved, and compared with a preparation method of the sucralose by taking high-purity sucralose-6-acetate as the raw materials through alkaline hydrolysis, the method provided in the invention has the advantages that the conversion rate of the raw materials and the yield of the sucralose are significantly improved. The present invention adopts a dual system of ethyl acetate and water, which can realize the mutual application of the dual systems for extraction and impurity removal, and enrich sucralose, so that sucralose can crystallize in large quantities in ethyl acetate, moreover, sucralose is recovered by means of repeated recycling, avoiding the loss of sucralose and greatly improving the overall yield of sucralose. Furthermore, compared with the hydrolysis in the traditional sodium methoxide/methanol system, the preparation method provided by the present invention has a lower production cost and is safer, more environmentally friendly.

### Brief Description of the Drawings

FIG. 1 is a flow chart of the preparation process for the sucralose refined product;
FIG. 2 is the flow chart of the preparation process for the sucralose crude product and the preparation of the sucralose crude product using the hydrolysis system.

### Detailed Description of Embodiments

The invention is further illustrated below with reference to examples and figures.

The invention provides a preparation method of a sucralose refined product, which comprises the following steps:
(1) Mixing the sucralose crude product with ethyl acetate, pulping, and then carrying out solid-liquid separation to obtain a pulped mother liquor and a pulped target product respectively; dissolving the pulped target product in water, and decoloring the obtained aqueous solution of the pulped target product by using activated carbon to obtain a pulped and decolored target product solution;
(2) Concentrating the pulped and decolored target product solution, and performing first crystallization on the obtained pulped and decolored target product concentrated solution to obtain a first crystallized target product and a primary crystallized mother liquor respectively;
(3) Dissolving the first crystallized target product in water, and carrying out second crystallization on the obtained first crystallized target product aqueous solution to obtain a second crystallized target product and a secondary crystallized mother liquor respectively; the secondary crystallized mother liquor is recycled to step (1) for dissolving the pulped target product;
(4) Dissolving the second crystallized target product in water, and performing solid-liquid separation on the obtained second crystallized target product aqueous solution after activated carbon decolorization to obtain a second crystallized decolorized target product aqueous solution and recovered activated carbon respectively; concentrating the second crystallized decolorized target product aqueous solution, and carrying out third crystallization on the obtained second crystallized decolorized target product concentrated solution to obtain a third crystallized target product and a tertiary crystallized mother liquor respectively; drying the third crystallized target product to obtain a sucralose refined product; recycling the tertiary crystallized mother liquor in step (3) for dissolving the first crystallized target product, and recycling the recovered activated carbon to step (1) for decoloring the pulped target product aqueous solution.

In the present invention, unless otherwise specified, all the raw material components are commercially available products well known to those skilled in the art.

In the present invention, the sucralose crude product is mixed with ethyl acetate, pulped, and then carried out solid-liquid separation to respectively obtain a pulped mother liquor and a pulped target product; the pulped target product is dissolved in water, the obtained pulped target product aqueous solution is mixed with activated carbon, and decolored to obtain a pulped and decolored target product solution; the pulped mother liquor is used for preparing sucralose crude product.

In the present invention, the sucralose crude product comprises the following components in percentage by mass: the proportion of the sucralose is preferably 85-95%, more preferably 88-92%; the content of sucralose-6-acetate is 0.5-1%, more preferably 0.6-0.9%; the ethyl acetate is preferably 1-3%, more preferably 1.5-2.5%; the content of the caramel-like impurities is preferably 1-13.5%, more preferably 5-10%. In the present invention, the ratio of the mass of the sucralose crude product to the volume of the ethyl acetate for pulping is preferably 1 kg: 1-1.5 L, more preferably 1 kg: 1.1-1.4 L, more preferably 1 kg: 1.3-1.4 L.

In the present invention, the pulping temperature is preferably 20-40 °C, more preferably room temperature. The pulping time is preferably 5-15 min, more preferably 10 min; the pulping is preferably carried out under stirring conditions. The solid-liquid separation method is not particularly limited in the invention, and a solid-liquid separation method known to those skilled in the art can be adopted, such as filtration, suction filtration or pressure filtration. In the present invention, the dissolving temperature is preferably 30-50 °C, more preferably 30-45 °C, further preferably 30-40 °C; the sugar degree (Bx) of the pulped target product aqueous solution is preferably 20-40%, more preferably 25-35%, further preferably 30%.

In the present invention, the activated carbon is preferably plant activated carbon, more preferably at least one of coconut shell activated carbon and shell activated carbon, the coal-based activated carbon can leave sulfur impurities and can influence the product taste of the sucralose, and the plant activated carbon adopted by the invention has low sulfur content and do not influence the product quality of sucralose. In the present invention, the mass of the activated carbon is preferably 0.5-1%, more preferably 0.6-0.9% of the dry weight of the sucralose crude product. In the present invention, the decolorizing temperature is preferably 40-50 °C, more preferably 45 °C, the decolorizing time is preferably 5-10 min, more preferably 6-8 min, and the decolorizing with activated carbon is to remove colored impurities in the sucralose crude product. After the activated carbon decoloration is completed, the invention preferably further comprises the step of carrying out solid-liquid separation on the obtained decolored system to obtain pulped decolored target product solution and activate carbon residues respectively. The solid-liquid separation method is not particularly limited in the invention, and a solid-liquid separation method known to those skilled in the art can be adopted, such as filtration, suction filtration or pressure filtration. In the present invention, the activated carbon residue is preferably subjected to solid waste treatment.

In the present invention, the method for preparing the crude sucralose preferably comprises the following steps:
(a) Extracting the raw material liquid by ethyl acetate to obtain a first ethyl ester phase and a first aqueous phase respectively; concentrating the first ethyl ester phase to obtain a first ethyl ester phase concentrate; the raw material liquid is an aqueous solution containing sucralose-6-acetate, sucralose diester and sucralose tetrachloride-6-acetate;
(b) Mixing and dissolving the first ethyl ester phase concentrate and water, and concentrating to obtain a sucralose-6-acetate aqueous solution;
(c) Mixing the sucralose-6-acetate aqueous solution with an alkali metal hydroxide for carrying out an alkaline hydrolysis reaction, neutralizing the obtained reaction solution, and filtering to obtain a second aqueous phase;
(d) Performing ethyl acetate extraction on the second aqueous phase to obtain a second ethyl ester phase and a third aqueous phase respectively;
(e) Washing the second ethyl ester phase with water to obtain a fourth aqueous phase and a third diethyl ester phase respectively; said fourth aqueous phase is recycled to step (b) for dissolving said first ethyl ester phase concentrate;
(f) Mixing the third ethyl ester phase with ethyl acetate, and carrying out azeotropic dehydration to obtain a third ethyl ester phase concentrate; mixing the third ethyl ester phase concentrate with ethyl acetate to obtain a fourth ethyl ester phase;
(g) Crystallizing the fourth ester phase to obtain a sucralose crude product and a fifth ethyl ester phase respectively;
(h) Washing the fifth ethyl ester phase with water to obtain a fifth aqueous phase and a sixth ethyl ester phase respectively; the fifth aqueous phase is recycled to step (b) for dissolving the first ethyl ester phase concentrate.

In the present invention, raw material liquid is extracted by ethyl acetate to obtain a first ethyl ester phase and a first aqueous phase respectively; the first ethyl ester phase is concentrated to obtain a first ethyl ester phase concentrate; the raw material liquid is an aqueous solution containing sucralose-6-acetate, sucralose diester and sucralose tetrachloride-6-acetate. The preparation method of the raw material solution is not particularly limited, and a preparation method for preparing a crude sucrose-6-acetate from sucrose as an initial raw material, which is well known to those skilled in the art, may be adopted, and specifically includes: preparing a solution containing sucrose-6-acetate by using sucrose as a raw material, N, N-Dimethylformamide (DMF) as a solvent, organotin as a catalyst and acetic anhydride as an acylating agent; and then sequentially carrying out chlorination (thionyl chloride) and ammonia neutralization on the obtained solution containing the sucrose-6-acetate, carrying out vacuum concentration until the solution is dry, and adding water for dissolution to obtain a raw material solution. In the invention, the content of the sucralose-6-acetate in the raw material liquid is preferably 50-80 g/L, and more preferably 55-75 g/L; the mass ratio of the sucralose-6-acetate, the sucralose diester, and the sucralose tetrachloride-6-acetate in the raw material liquid is preferably 1: 0.06-0.15: 0.06-0.15; the raw material liquid preferably also comprises NH₄Cl and organic impurities, and the concentration of NH₄Cl is preferably 80-150 g/L, and more preferably 80-100 g/L; the concentration of the organic impurities is preferably 30-80 g/L, and more preferably 30-50 g/L. In the invention, the temperature of ethyl acetate extraction is preferably 40-60 °C, more preferably 45-55 °C, and further preferably 50 °C; the number of times of ethyl acetate extraction is not particularly limited, and the sucralose-6-acetate content in an aqueous phase (namely a first aqueous phase) obtained by the last time of ethyl acetate extraction is less than 0.1 g/L, specifically 5-8 times; the time for single ethyl acetate extraction is preferably 10-30 min, and more preferably 15-20 min; the volume ratio of the raw material liquid to ethyl acetate for single ethyl acetate extraction is preferably 1: 0.2-0.5, more preferably 1: 0.3-0.4. In the invention, ethyl ester phases obtained by ethyl acetate extraction are combined into a first ethyl ester phase, and a raffinate phase obtained by the last ethyl acetate extraction is a first aqueous phase, wherein the first aqueous phase is preferably subjected to high-salt wastewater treatment. According to the method, hot ethyl acetate (40-60 °C) is adopted to extract the raw material liquid, so that the using amount of the ethyl acetate can be reduced, and meanwhile, the dissolving amount of fat-soluble impurities is increased, so that the subsequent solubility of sucralose in the ethyl acetate is increased, and the crystal sugar degree and the sucralose crystallization yield are further improved. The concentration method is not particularly limited in the invention, and the concentration method known to a person skilled in the art can be adopted, specifically, for example, vacuum concentration, the concentration temperature is preferably 60-80 °C, more preferably 65-75 °C, the vacuum degree of the concentration is preferably -0.1 to -0.08 MPa (gauge pressure), more preferably -0.09 to -0.08 MPa (gauge pressure), the concentration time is not particularly limited in the invention, and the concentration is carried out until the ethyl acetate content in the obtained first ethyl ester phase concentrate is < 0.5 g/L. According to the method, the residual quantity of the ethyl acetate in the first ethyl ester phase concentrate is controlled, so that byproducts such as acetic acid and ethanol generated by the ethyl acetate in the subsequent alkaline hydrolysis step can be avoided, and the purity and the yield of the sucralose are further improved.

After the first ethyl ester phase concentrate is obtained, the first ethyl ester phase concentrate is mixed with water for dissolving, and then the mixture is concentrated to obtain the sucralose-6-acetate aqueous solution. In the present invention, the volume ratio of the raw material liquid to water is preferably 1: 0.5-1, more preferably 1: 0.6-0.8. The concentration method is not particularly limited in the invention, and the concentration method known to a person skilled in the art can be adopted, specifically, the concentration temperature is preferably 60-80 °C, more preferably 65-75 °C, the vacuum degree of the concentration is preferably -0.1 to -0.08 MPa (gauge pressure), more preferably -0.09 to -0.08 MPa (gauge pressure), the concentration time is not particularly limited in the invention, and the concentration is carried out as long as the content of ethyl acetate in the obtained sucralose-6-acetate aqueous solution is concentrated to be < 0.5 g/L. According to the method, the residual quantity of the ethyl acetate in the sucralose-6-acetate aqueous solution is controlled, so that byproducts such as acetic acid and ethanol generated by the ethyl acetate in the subsequent alkaline hydrolysis step can be avoided, and the purity and the yield of the sucralose are further improved. In the present invention, the pulped mother liquor is preferably recycled for use in preparing the sucralose crude product, and more preferably for dissolving the first ethyl ester phase concentrate.

After the sucralose-6-acetate aqueous solution is obtained, the sucralose-6-acetate aqueous solution is mixed with an alkali metal hydroxide for carrying out an alkaline hydrolysis reaction, and the obtained reaction solution is neutralized and then filtered to obtain a second aqueous phase. In the present invention, the alkali metal hydroxide preferably includes sodium hydroxide and/or potassium hydroxide; the alkali metal hydroxide is preferably used in the form of an aqueous alkali metal hydroxide solution, and the concentration of the aqueous alkali metal hydroxide solution is preferably 10-40 wt%, and more preferably 20-35 wt%; the dosage of the alkali metal hydroxide is not particularly limited, and the **pH** value in the alkaline hydrolysis reaction process can be ensured to be 11-13, more preferably 11.5-12.5, and further preferably 12; the temperature of the alkaline hydrolysis reaction is preferably 0-10 °C, more preferably 0-8 °C, and further preferably 1-5 °C; the time of the alkaline hydrolysis reaction is preferably 3-6 h, more preferably 3.5-5.5 h, and further preferably 4-5 h. The invention carries out the alkaline hydrolysis reaction under the conditions, the sucralose diester can be hydrolyzed to generate sucralose, the sucralose tetrachloride-6-acetate can be dechlorinated and hydrolyzed to generate sucralose, and by-products generated by overhigh **pH** value of the alkaline hydrolysis reaction or overhigh temperature of the alkaline hydrolysis reaction can be avoided. Compared with the method using pure sucralose-6-acetate as a raw material, the method has the advantages that the yield of the sucralose is significantly improved, and the aqueous solution of the crude sucralose-6-acetate is not required to be purified, so that the process flow is greatly shortened, and the production cost is reduced. In the present invention, the acid for neutralization preferably comprises hydrochloric acid, and the concentration of the hydrochloric acid is preferably 15-35 wt%, and more preferably 20-30 wt%. The using amount of the acid is not particularly limited, and the system can be neutralized to a **pH** value of 6.8-7. In the invention, the temperature of neutralization and filtration is preferably 50-70 °C, more preferably 55-65 °C and further preferably 60 °C independently. After the alkaline hydrolysis is completed, viscous substances will be generated, and a large amount of sucralose will be mixed in these viscous substances, in the present invention, the reaction solution obtained from the alkaline hydrolysis reaction is heated to 50-70 °C for neutralization, which can dissolve the sucralose mixed in the viscous substances, then, hot filtration can be carried out to remove insoluble substances such as carbon residues and tar, and simultaneously it can also avoid the coking of sucralose caused by overly high temperature.

After the second aqueous phase is obtained, the second aqueous phase is subjected to ethyl acetate extraction to obtain a second ethyl ester phase and a third aqueous phase respectively. In the invention, the number of times of ethyl acetate extraction is preferably 4-7; the volume ratio of the second aqueous phase to the ethyl acetate used for the single extraction is preferably 1:1-3, more preferably 1:1.5- 2.5; in the invention, ethyl ester obtained by the first to second ethyl acetate extraction is preferably combined to be used as a second ethyl ester phase; preferably, the ethyl acetate phase obtained by the third to the seventh ethyl acetate extraction is used for ethyl acetate extraction of the second aqueous phase in the preparation process of the next batch of sucralose crude product, specifically, the ethyl acetate phase obtained by the third ethyl acetate extraction is used for ethyl acetate extraction of the first aqueous phase in the preparation process of the next batch of sucralose crude product, the ethyl acetate phase obtained by the fourth ethyl acetate extraction is used for ethyl acetate extraction of the second aqueous phase in the preparation process of the next batch of sucralose crude product, the ethyl acetate phase obtained by the fifth ethyl acetate extraction is used for ethyl acetate extraction of the second aqueous phase in the preparation process of the next sucrose crude product, the ethyl acetate phase obtained by the sixth ethyl acetate extraction is used for ethyl acetate extraction of the fourth time of the second aqueous phase in the preparation process of the next batch of sucralose crude product, and the ethyl acetate phase obtained by the seventh ethyl acetate extraction is used for the fifth ethyl acetate extraction of the second aqueous phase in the next sucrose crude product (namely, the ethyl acetate phase obtained by the third to the seventh ethyl acetate extraction is sequentially used for the first to the fifth ethyl acetate extraction of the second aqueous phase in the next batch of sucralose crude product preparation process), the sixth to the seventh ethyl acetate extraction of the second aqueous phase in the next batch of sucralose crude product preparation process is preferably performed by using pure ethyl acetate. In the present invention, the sucralose content in said third aqueous phase is preferably < 0.5 g/L.

After the second ethyl ester phase is obtained, the second ethyl ester phase is washed by water to obtain a fourth aqueous phase and a third diethyl ester phase respectively; the fourth aqueous phase is recycled to step (b) for dissolving the first ethyl ester phase concentrate. In the invention, the washing times are preferably 3-6 times; the volume ratio of the second ethyl ester phase to the water used for single-time is preferably 1: 0.1-0.3, more preferably 1: 0.15-0.25; preferably, the aqueous phase obtained by the first water washing is used as a fourth aqueous phase, preferably, the aqueous phase obtained by the second to the sixth water washing is used for the water washing of the second ethyl ester phase in the next batch of sucralose crude product preparation process, specifically, the aqueous phase obtained by the second water washing is used for the first water washing of the second ethyl ester phase in the next batch of sucralose crude product preparation process, the aqueous phase obtained by the third water washing is used for the second water washing of the second ethyl ester phase in the next batch of sucralose crude product preparation process, the aqueous phase obtained by the fourth water washing is used for the third water washing of the second ethyl ester phase in the next batch of sucralose crude product preparation process, the aqueous phase obtained by the fifth water washing is used for the fourth water washing of the second ethyl ester phase in the next batch of sucralose crude product preparation process, and the aqueous phase obtained by the sixth water washing is used for the fifth water washing of the second ethyl ester phase in the next batch of sucralose crude product preparation process (namely, the aqueous phases obtained by the second to the sixth water washing are sequentially used for the first to the fifth water washing of the third ethyl ester phase in the next batch of sucralose crude product preparation process), and the sixth water washing of the second ethyl ester phase of the next batch of sucralose crude product is preferably performed by using pure water.

After the third ethyl ester phase is obtained, mixing the third ethyl ester phase with ethyl acetate, and performing azeotropic dehydration to obtain a third ethyl ester phase concentrate; mixing the third ester phase concentrate with ethyl acetate to obtain a fourth ester phase. The concentration method is not particularly limited in the present invention, and a concentration method known to those skilled in the art may be adopted, specifically, vacuum concentration is adopted, the concentration temperature is preferably 60-80 °C, more preferably 65-75 °C, the vacuum degree of the concentration is preferably -0.1 to -0.08 MPa (gauge pressure), more preferably -0.09 to -0.08 MPa (gauge pressure), the concentration time is not particularly limited in the present invention, as long as the concentration is carried out until the water content of the third ethyl ester phase concentrate is < 0.5 wt%. The amount of the ethyl acetate is not particularly limited, and the sugar degree of the fourth ethyl ester phase is 40- 60%, and more preferably 45-55%.

After the fourth ester phase is obtained, the fourth ester phase is crystallized to obtain a sucralose crude product and a fifth ester phase respectively. In the invention, the crystallization temperature is preferably 40-60 °C, and more preferably 45-55 °C; the crystallization time is preferably 12-30 h, and more preferably 15-25 h. The solubility of the sucralose in the ethyl acetate is low, the fat-soluble caramel impurities in the system are introduced into the ethyl acetate in the process of recycling the fourth aqueous phase and the fifth aqueous phase, so that the solubility of the sucralose in the ethyl acetate can be significantly increased, and the impurities in the system are balanced in the methods of extraction, water washing and recycling, so that the sucralose is enriched in the ethyl acetate and crystallized. Moreover, the crystallization of sucralose under the temperature condition has the following two advantages: firstly, more sucralose can be obtained without being mixed with more fat-soluble impurities; secondly, the sucralose will not be coked due to higher crystallization temperature, thereby improving the yield of the sucralose. After the crystallization is completed, the method preferably further comprises solid-liquid separation to obtain a sucralose crude product and a fifth ethyl ester phase respectively. The solid-liquid separation method is not particularly limited in the invention, and a solid-liquid separation method known to those skilled in the art can be adopted, such as filtration, suction filtration or pressure filtration.

After a fifth ethyl ester phase is obtained, washing the fifth ethyl ester phase with water to obtain a fifth aqueous phase and a sixth ethyl ester phase respectively; the fifth aqueous phase is recycled to step (b) for dissolving the first ethyl ester phase concentrate. The number of times of water washing is not particularly limited, as long as the sucralose content in the sixth ethyl ester phase obtained by the last water washing is < 0.1g/L, specifically 4-8 times. In the present invention, the volume ratio of the fifth ethyl ester phase to the water used for single-time water washing is preferably 1: 0.5-1, more preferably 1: 0.6-0.8. In the invention, the sixth ethyl ester phase is preferably concentrated to obtain recovered ethyl acetate and sugar residues, and the sugar residues are preferably subjected to solid waste treatment. After crystallization is completed, the amount of fat-soluble impurities in the fifth ethyl ester phase is relatively large, and the solubility of sucralose in water is relatively high, the method of water washing adopted by the present invention can recover the uncrystallized sucralose into the fifth water phase, and recycle the fifth water phase, thereby improving the yield and recovery reuse of sucralose, at the same time, it can also remove the fat-soluble impurities in sucralose crystals and improve its purity.

The water-soluble impurities and the fat-soluble impurities are generated in a series of reaction processes of the sucrose, so that they have a similar main body structure with sucralose, and the water-soluble impurities, the fat-soluble impurities and sucralose have a certain mutual solubility. The invention uses the relationship between the three, and selects water and ethyl acetate as the solvent for removing water-soluble impurities and fat-soluble impurities, thereby using two solvents to dissolve and carry sucralose, realizing the exchange of sucralose in the two solvents, enriching sucralose in ethyl acetate, and crystallizing from sucralose to obtain a sucralose crude product. Specifically, the solubility of sucralose in ethyl acetate is much less than that in water, so that more fat-soluble impurities are retained in the system during pretreatment. After the alkaline hydrolysis reaction, extracting sucralose from the alkaline hydrolysis solution by using multiple times and multiple volumes of ethyl acetate. At this time, the concentration of sucralose in ethyl acetate obtained by multiple extractions shows a gradient decrease phenomenon, the sucralose content in the ethyl acetate phase obtained by the previous two extractions is higher and is used as a raw material for sucralose crystallization. The remaining batch of ethyl acetate phase is used for extraction of the next batch of alkaline hydrolysis solution, so that a saturated ethyl sucralose acetate solution can be obtained. The content of water-soluble impurities affects the crystallization of sucralose in the ethyl acetate phase, so the ethyl acetate phase containing sucralose in the previous two times needs a small amount of pure water for washing for a plurality of times, thereby removing the water-soluble impurities. The concentration of sucralose in the aqueous phase obtained by water washing also decreases with the increase in the number of water washing times, in order to recover this part of sucralose, the water washing liquid (fourth aqueous phase) which is washed for the first time and contains the highest sucralose concentration is recycled to be mixed and dissolved with the first ethyl ester phase concentrate, and the rest water washing liquid is used for washing the second ethyl ester phase of the next batch of sucralose. The concentration and drying treatment of the obtained third ethyl ester phase after water washing aims to remove the water in the system, because the residual water can increase the difficulty of crystallization of the sucralose in the ethyl acetate. After concentration and drying, it is still necessary to add fresh ethyl acetate to continue the concentration and drying process, the purpose is to completely remove the water, at this time, the third ethyl ester concentrate is dissolved with fresh ethyl acetate to a specific sugar degree, and the obtained fourth ethyl ester phase is crystallized to obtain a sucralose crude product.

After obtaining the pulped and decolored target product solution, concentrating the pulped and decolored target product solution, and carrying out first crystallization on the obtained pulped and decolored target product concentrated solution to respectively obtain a first crystallized target product and a primary crystallized mother liquor. In the invention, the concentration temperature is preferably 60-80 °C, and more preferably 65-75 °C; the vacuum degree of the concentration is preferably -0.1 to -0.08 MPa (gauge pressure), and more preferably -0.09 to -0.08 MPa (gauge pressure); the concentration time is not particularly limited, and the concentration is carried out until the sugar degree of the pulped and decolored target product concentrated solution is 40-65%, wherein the sugar degree is more preferably 45-60%, and is further preferably 50-55%; the invention carries out concentration under the above conditions, and can avoid high-temperature coking of sucralose; the sugar degree of the pulped and decolored target product concentrated solution is too high or too low and is not easy to crystallize. In the present invention, the temperature of the first crystallization is preferably room temperature, and the time of the first crystallization is preferably 8-16 h, more preferably 10-15 h, and further preferably 12-13 h. After the first crystallization is completed, the present invention preferably further comprises subjecting the obtained first crystallization system to solid-liquid separation to obtain a first crystallized target product and a primary crystallized mother liquor respectively. The solid-liquid separation method is not particularly limited in the invention, and a solid-liquid separation method known to those skilled in the art can be adopted, such as filtration, suction filtration or pressure filtration. In the present invention, the primary crystallized mother liquor is preferably recycled for preparing the sucralose crude product, and more preferably used for azeotropic water removal after being mixed with the third ethyl ester.

After the first crystallized target product is obtained, dissolving the first crystallized target product in water, and carrying out the second crystallization on the obtained first crystallized target product aqueous solution to obtain a second crystallized target product and a secondary crystallized mother liquor respectively; recycling the secondary crystallized mother liquor to step (1) for dissolving the pulped target product. In the invention, the dissolving temperature is preferably 40-60 °C, more preferably 45-55 °C, and further preferably 50 °C. In the present invention, the sugar degree of the first crystallized target product aqueous solution is preferably 40-65%, more preferably 45-60%, and further preferably 50-55%. In the invention, the temperature of the second crystallization is preferably room temperature, and the time of the second crystallization is preferably 8-16 h, more preferably 10-15 h, and further preferably 12-13 h. After the second crystallization is completed, the method preferably further comprises the step of carrying out solid-liquid separation on the obtained second crystallization system to obtain a second crystallized target product and a secondary crystallized mother liquor respectively. The solid-liquid separation method is not particularly limited in the invention, and a solid-liquid separation method known to those skilled in the art can be adopted, such as filtration, suction filtration or pressure filtration.

After the second crystallized target product is obtained, dissolving the second crystallized target product in water, and performing solid-liquid separation on the obtained second crystallized target product aqueous solution after activated carbon decolorization to obtain a second crystallized decolorized target product aqueous solution and recovered activated carbon respectively; concentrating the second crystallized decolorized target product aqueous solution, and carrying out third crystallization on the obtained second crystallized decolorized target product concentrated solution to obtain a third crystallized target product and a tertiary crystallized mother liquor respectively; drying the third crystallized target product to obtain a sucralose refined product; recycling the tertiary crystallized mother liquor to step (3) for dissolving the first crystallized target product, and recycling the recovered activated carbon to step (1) for decoloring the pulped target product aqueous solution. In the invention, the dissolving temperature is preferably 40-60 °C, more preferably 45-55 °C, and even more preferably 50 °C. In the present invention, the sugar degree of the second crystallized target product aqueous solution is preferably 20-30%, more preferably 22-28%, and further preferably 24-26%. In the invention, the activated carbon is preferably plant activated carbon, more preferably at least one of coconut shell activated carbon and shell activated carbon, the coal-based activated carbon can leave sulfur impurities and can influence the product taste of the sucralose, and the plant activated carbon adopted by the invention has low sulfur content and do not influence the product quality of sucralose. In the invention, the mass of the activated carbon is preferably 0.5-1%, more preferably 0.6-0.9%, and further preferably 0.7-0.8% of the dry weight of the sucralose crude product; theactivated carbon decoloring temperature is preferably 40-50 °C, more preferably 45 °C, theactivated carbon decoloring time is preferably 5-10 min, more preferably 6-8 min, and the purpose of activated carbon decoloring is to remove residual water-soluble impurities and improve the purity and quality of sucralose refined products. The solid-liquid separation method is not particularly limited in the invention, and a solid-liquid separation method known to those skilled in the art can be adopted, such as filtration, suction filtration or pressure filtration. In the present invention, the temperature of the concentration is preferably 60-80 °C, more preferably 65-75 °C, the vacuum degree of the concentration is preferably -0.1 to -0.08 MPa (gauge pressure), more preferably -0.09 to -0.08 MPa (gauge pressure), the time of the concentration is not particularly limited, as long as the concentration is performed until the sugar degree of the second crystal decolorized target product concentrate obtained is 40-65%, more preferably 45-60%, and even more preferably 50-55%. In the invention, the temperature of the third crystallization is preferably 30-50 °C, more preferably 35-45 °C, and the time of the third crystallization is preferably 4-12 h, more preferably 5-10 h. After the third crystallization is completed, the method preferably further comprises the step of carrying out solid-liquid separation on the obtained crystallization system to obtain a third crystallized target product and a third crystallized mother liquor respectively. The solid-liquid separation method is not particularly limited in the invention, and a solid-liquid separation method known to those skilled in the art can be adopted, such as filtration, suction filtration or pressure filtration. In the invention, the drying temperature is preferably 40-50 °C, more preferably 45 °C, and the drying time is preferably 8-12 h, more preferably 10 h.

The technical solution of the present invention will be clearly and completely described below with reference to the embodiments of the present invention. It should be apparent that the described embodiments are only some embodiments of the present invention, and not all embodiments. All other embodiments, which can be obtained by a person skilled in the art without making any creative effort based on the embodiments in the present invention, belong to the protection scope of the present invention.

The contents of each substance in the following examples and comparative examples were measured by High Performance Liquid Chromatography (HPLC) using an external standard method under the following conditions, analytical measurement conditions of HPLC: Japanese Shimadzu high performance liquid chromatograph, with RID-10A refractive index detection unit, LC-10ADVP high-pressure pump, CTO-10ASVP constant temperature box; chromatographic column: Agilent XDB C18 column (250 mm×4.6 mm, 5 µm); mobile phase: methanol-0.125 wt% aqueous dipotassium hydrogen phosphate (4:6, v/v); column temperature: 40 °C; mobile phase flow rate: 1.0 mL/min; wherein, methanol (chromatogram purity), dipotassium hydrogen phosphate (analytical purity), water is ultrapure water.

Method for preparing the raw material liquid (denoted as first aqueous solution) used in the following examples and comparative examples: using sucrose as raw material, DMF as solvent, organotin as catalyst, acetic anhydride as acylating agent to prepare a solution containing sucrose-6-acetate; and then sequentially carrying out chlorination (thionyl chloride) and ammonia neutralization on the obtained solution containing the sucrose-6-acetate, carrying out vacuum concentration to dryness, and adding water to dissolve to obtain the raw material solution.

### Example 1

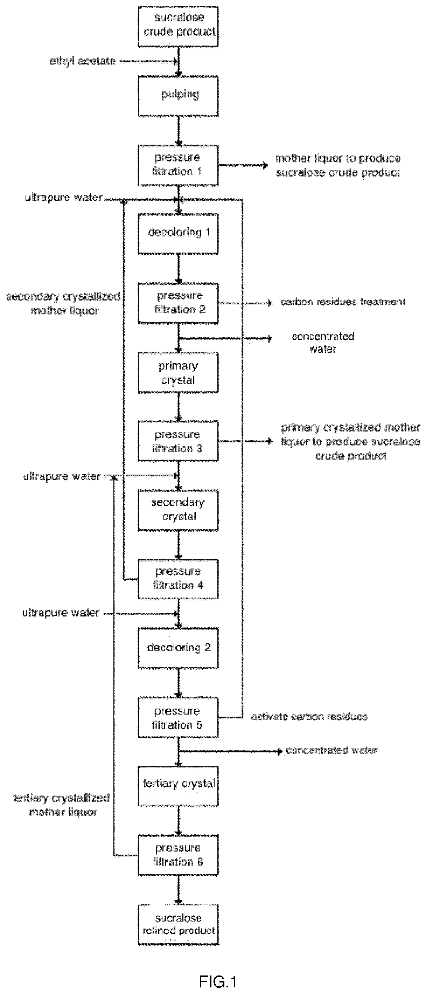

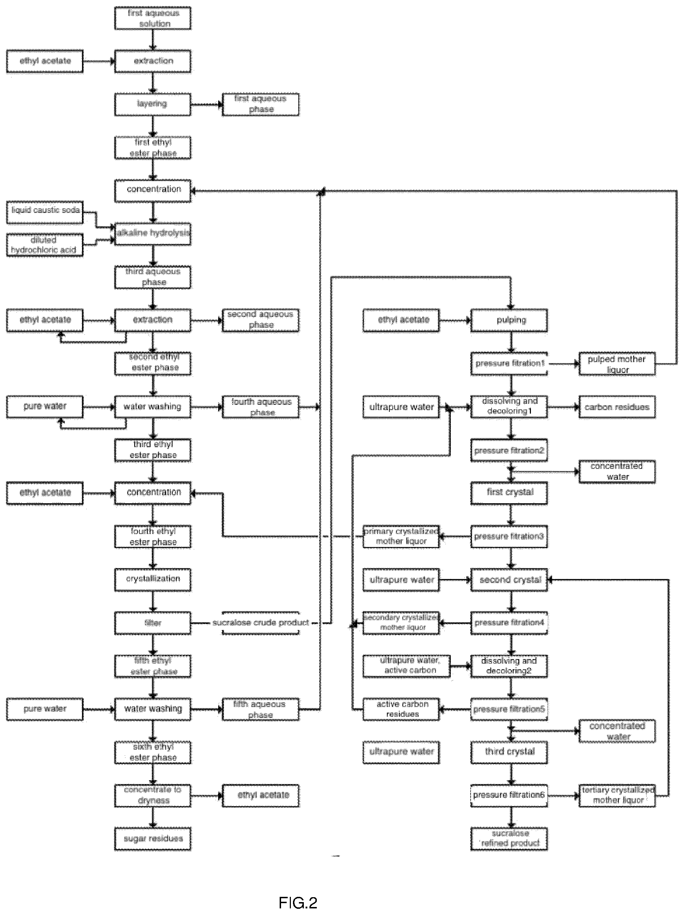

The process flow charts shown in the figures 1-2 are adopted to prepare the sucralose refined product, and the specific steps are as follows:
(1) Extraction step: adding 0.9 L of ethyl acetate into 3 L of first aqueous solution, stirring and extracting for 15 min at 50 °C, carrying out phase separation to obtain an organic phase and a aqueous phase, repeating the extraction step for 6 times on the aqueous phase, combining ester phases after the extraction is finished to obtain a first ethyl ester phase, and carrying out the last extraction to obtain the aqueous phase which is the first aqueous phase (the content of sucralose-6-acetate is 0.07 g/L, and the first aqueous phase is subjected to high-salt wastewater treatment). Wherein the composition of the first aqueous solution is shown in table 1.

**Table 1. Composition of the first aqueous solution**

| Components | Content |
|---|---|
| Sucralose-6-acetate | 55.2 g/L |
| Sucralose diester | 5.0 g/L |
| Tetrachlorosucrose-6-acetate | 5.3 g/L |
| NH₄Cl | 81.7 g/L |
| Other organic impurities | 33.5 g/L |
| Water | 81.4% |

(2) Concentration and drying step: concentration and drying were carried out on the first ethyl ester phase under vacuum at 80 °C, -0.08MPa (abbreviated as concentrated to dryness), and water (1800 mL) was added to the resulting first ethyl ester phase concentrate, followed by concentration to 1.8 L under the above conditions to obtain an aqueous solution of sucralose-6-acetate (ethyl acetate content 0.40 g/L), which was then transferred to a three-necked flask.
(3) Alkaline hydrolysis step: cooling the obtained sucralose-6-acetate aqueous solution to 1 °C, then dropwise adding a 32 wt% sodium hydroxide aqueous solution), uniformly mixing, carrying out alkaline hydrolysis at 1 °C for 5 h, keeping the pH value of the system to be 12.2 in the alkaline hydrolysis process, heating to 50 °C after the alkaline hydrolysis is finished, then dropwise adding 30 wt% diluted hydrochloric acid to adjust the pH value to be 7.5, and filtering to obtain a second aqueous phase (2 L, the content of the sucralose-6-acetate is 0.04 g/L) and filter residues (solid waste treatment).
(4) Separation step: adding 4 L of ethyl acetate into the second aqueous phase, stirring and extracting for 15 min at 50 °C, repeating the extraction operation on the obtained aqueous phase, extracting for 6 times totally, combining ester phases obtained by the first to the second extraction to obtain a second ethyl ester phase (8.6 L), and sequentially using the ester phases obtained by the third to the sixth ethyl acetate extraction in the first to the fourth ethyl acetate extraction of the second aqueous phase in the preparation process of the next batch of sucralose crude products. Adding 1.6 L of water into the second ethyl ester phase for washing for 6 times, wherein the aqueous phase obtained by the first washing is a fourth aqueous phase, and the fourth aqueous phase is used in step (2) instead of water to mix with the first ethyl ester phase concentrate; and sequentially using the aqueous phase obtained by the second to the sixth water washing for the first to the fifth water washing of the second ethyl ester phase in the preparation process of the next batch of sucralose crude product, wherein the ester phase obtained after the water washing is the third ethyl ester phase. Concentrating and drying the third ester phase under the condition of 70 °C, -0.1 MPa (gauge pressure), adding ethyl acetate (adding 3 times, and 0.9 L is added every time) in the concentration and drying process to dehydrate so as to ensure that the moisture is completely removed, and adding ethyl acetate into the obtained third ester phase concentrate to adjust the sugar degree Bx to be 45% to obtain the fourth ester phase.
(5) Crystallization step: crystallizing the fourth ester phase at 45 °C for 24 h, and performing suction filtration to obtain a sucralose crude product and 1.3L of a fifth ester phase respectively (after 4 times of balance); washing the fifth ethyl ester phase for 6 times (the water consumption is 0.6L each time), wherein the ester phase after washing is a sixth ester phase (the content of sucralose is 0.02 g/L), and carrying out concentration and drying on the sixth ester phase to obtain recovered ethyl acetate and sugar residue respectively; the aqueous phases were combined as a fifth aqueous phase which was used in step (2) instead of water to mix with the first ethyl ester phase concentrate.
(6) Pulping and decoloring step: adding ethyl acetate into the sucralose crude product, pulping for 10min at room temperature, filtering to obtain a pulped mother liquor and a pulped target product respectively, and recycling the pulped mother liquor to step (2) for dissolving the first ethyl ester phase concentrate; dissolving the pulped target product in water at 45 °C, adding coconut shell activated carbon into the obtained pulped target product aqueous solution (the sugar degree is 20%), and decoloring at 40 °C for 10min to obtain pulped decolored target product solution; wherein, the ratio of the dry weight of the sucralose crude product to the volume of the ethyl acetate is 1 kg: 1 L; the mass of the coconut shell activated carbon is 0.5% of the dry weight of the sucralose crude product.
(7) First crystallization step: concentrating the pulped decolorized target product solution at 80 °C and -0.08 MPa (gauge pressure), carrying out first crystallization on the obtained pulped decolorized target product concentrated solution (the sugar degree is 40%) at room temperature for 16 h, and filtering to obtain a first crystallized target product and a primary crystallized mother liquor respectively; mixing the primary crystallized mother liquor with the third ethyl ester in step (4) and then carrying out azeotropic dehydration.
(8) Second crystallization step: dissolving the first crystallized target product in water at 60 °C, and carrying out second crystallization on the obtained first crystallized target product aqueous solution (the sugar degree is 65%) for 8 h at room temperature to obtain a second crystallized target product and a secondary crystallized mother liquor respectively; recycling the secondary crystallized mother liquor to step (6) for dissolving the pulped target product.
(9) Third crystallization step: dissolving the second crystallized target product in water at 50 °C, adding coconut shell activated carbon (the mass of the activated carbon is 1% of the dry weight of the sucralose crude product) into the obtained second crystallized target product aqueous solution (the sugar degree is 25%), decoloring for 5 min at 45 °C, and filtering to obtain a second crystallized decolored target product aqueous solution and recovered activated carbon respectively; concentrating the second crystallized decolorized target product aqueous solution at 60 °C, -0.1 MPa (gauge pressure), and carrying out third crystallization on the obtained second crystallized decolorized target product concentrated solution ( the sugar degree is 65%) at 50 °C for 4 h to obtain a third crystallized target product and a third crystallized mother liquor respectively; drying the third crystallized target product to obtain a sucralose refined product; recycling the tertiary crystallized mother liquor to step (3) for dissolving the first crystallized target product, and recycling the recovered activated carbon to step (1) for decoloring pulped target product aqueous solution;
(10) Recycling step: according to the operations of the steps (1) to (9) (recorded as recycling 0 time), recycling 17 times the fourth aqueous phase, the fifth aqueous phase, the aqueous phases obtained by the second to the sixth water washing, and the ester phases obtained by the third to the sixth ethyl acetate extraction of the second aqueous phase, wherein the modification of step (2) of "adding water (1800 mL) into the obtained first ethyl ester phase concentrate for mixing" is that "the obtained first ethyl ester phase, the fourth aqueous phase, the fifth aqueous phase and the pulped mother liquor are mixed"; In step (4), replacing "adding ethyl acetate into the obtained third ethyl ester phase concentrate to adjust the sugar degree Bx to be 43%" with "adding the primary crystallized mother liquor into the obtained third ethyl ester phase concentrate, concentrating at 80 °C and-0.08 MPa (gauge pressure) until water content is 0.4 wt%, then adding ethyl acetate to adjust the sugar degree Bx to be 43%", wherein the first to fourth ethyl acetate extraction of the second aqueous phase respectively utilizes the ester phases obtained by the third to the sixth ethyl acetate extraction of the second aqueous phase in the preparation process of the previous batch of sucralose crude product, the fifth to the sixth ethyl acetate extraction of the second aqueous phase utilizes pure ethyl acetate, the first to the fifth water washing of the second ethyl ester phase sequentially utilizes the second to the sixth water washing of the second ethyl ester phase in the preparation process of the previous batch of sucralose crude product, the sixth water washing of the second ethyl ester phase utilizes pure water; in step (6), replacing "placing the pulped target product in water for dissolving at 60 °C" with "placing the pulped target product in water and the secondary crystallized mother liquor for dissolving at 60 °C", and in step (6), replacing the coconut shell activated carbon with the recycled activated carbon obtained in step (9); in step (8), replacing "placing the first crystallized target product in water" with "placing the first crystallized target product in water and the tertiary crystallized mother liquor". The purity and yield data of the sucralose refined product are shown in table 2.

**Table 2. Purity and yield of sucralose refined product**

| recycling times | mass of crude product/g | purity/% | yield/% | average yield/% |
|---|---|---|---|---|
| 0 | 42.38 | 99.8 | 28.24 | 70.57 |
| 1 | 95.07 | 99.7 | 63.28 | |
| 2 | 133.25 | 99.8 | 88.78 | |
| 3 | 152.91 | 99.9 | 101.99 | |
| 4 | 168.36 | 99.8 | 112.17 | 114.96 |
| 5 | 173.29 | 99.7 | 115.34 | |
| 6 | 174.70 | 99.7 | 116.28 | |
| 7 | 173.19 | 99.8 | 115.39 | |
| 8 | 167.24 | 99.6 | 111.20 | |
| 9 | 175.75 | 99.7 | 116.98 | |
| 10 | 171.77 | 99.9 | 114.56 | |
| 11 | 175.43 | 99.8 | 116.88 | |
| 12 | 175 44 | 99.7 | 116.77 | |
| 13 | 172.78 | 99.6 | 114.89 | |
| 14 | 172.58 | 99.7 | 114.87 | |
| 15 | 168.09 | 99.8 | 111.99 | |
| 16 | 173.33 | 99.6 | 115.25 | |
| 17 | 175.66 | 99.7 | 116.92 | |

| | | | | |
|---|---|---|---|---|
| Note: yield=mass of sucralose/mass of sucralose-6-acetate completely converted to sucralose ×100%. In the first aqueous solution, both the sucralose diester and the sucralose tetrachloride-6-acetate can be converted into sucralose after alkaline hydrolysis, and the theoretical maximum yield in example 1 is 117.48%. | | | | |

As can be seen from Table 2, the mother liquors need to be recycled for at least 4 times to achieve stable yield of sucralose, and the yield is still over 116% after 17 times of recycling.

### Example 2

The sucralose refined product was prepared according to the method of example 1, which differs from example 1 in that:
(1) Extraction step: adding 1.2 L of ethyl acetate into 3L of first aqueous solution, stirring and extracting for 10min at 45 °C, carrying out phase separation to obtain an organic phase and an aqueous phase, repeating the extraction step for 6 times on the aqueous phase, combining ester phases after the extraction is finished to obtain a first ethyl ester phase, and the aqueous phase obtained by the last extraction is the first aqueous phase (the content of sucralose-6-acetate is 0.05 g/L, and the first aqueous phase is subjected to high-salt wastewater treatment), wherein the main components of the used first solution are shown in Table 3.

**Table 3. Composition of the first solution**

| Components | Content |
|---|---|
| Sucralose-6-acetate | 51.4 g/L |
| Sucralose diester | 5.7 g/L |
| Tetrachlorosucrose-6-acetate | 4.8 g/L |
| NH₄Cl | 84.6 g/L |
| Other organic impurities | 35.1 g/L |
| Water | 82.7% |

(2) Concentration and drying step: the first ethyl ester phase was concentrated and dried under vacuum at 80 °C, -0.08 MPa(abbreviated as concentrated to dryness), and water (1800 mL) was added to the resulting first ethyl ester phase concentrate, and concentrated to 1.7 L under the above conditions to obtain an aqueous solution of sucralose-6-acetate (the content of ethyl acetate is 0.26 g/L), which was then transferred to a three-necked flask.
(3) Alkaline hydrolysis step: cooling the obtained sucralose-6-acetate aqueous solution to 2 °C, then dropwise adding a 32 wt% sodium hydroxide aqueous solution), uniformly mixing, carrying out alkaline hydrolysis for 5 h at 2 °C, keeping the pH value of the system to be 12.1 in the alkaline hydrolysis process, heating to 50 °C after the alkaline hydrolysis is finished, then dropwise adding 30 wt% diluted hydrochloric acid to adjust the pH value to be 7, and filtering to obtain a second aqueous phase (1.90 L, the content of sucralose-6-acetate is 0.01 g/L) and filter residues (solid waste treatment).
(4) Separation step: adding 4 L of ethyl acetate into the second aqueous phase, stirring and extracting for 10 min at 45 °C, repeating the extraction operation on the obtained aqueous phase, extracting for 6 times totally, combining ester phases obtained by the first to the second extraction as a second ethyl ester phase (8.5 L), and sequentially using the ester phases obtained by the third to the sixth extraction for the first to fourth ethyl acetate extraction of the second aqueous phase in the preparation process of the next batch of sucralose crude products. Adding 1.8 L of water into the second ethyl ester phase for washing for 6 times, wherein the aqueous phase obtained by the first washing is a fourth aqueous phase, and the fourth aqueous phase is used in step (2) instead of water to mix with the first ethyl ester phase concentrate; and sequentially using the aqueous phase obtained by the second to the sixth water washing for the first to the fifth water washing of the second ethyl ester phase in the preparation process of the next batch of sucralose crude product, wherein the ester phase obtained after the water washing is the third ethyl ester phase. concentrating and drying the third ester phase at 75 °C and under the condition of -0.1 MPa (gauge pressure), adding ethyl acetate (add 3 times, and 0.8 L is added every time) in the concentration and drying process to dehydrate so as to ensure that the moisture is completely removed, and adding ethyl acetate into the obtained third ester phase concentrate to adjust the sugar degree Bx to be 44% so as to obtain a fourth ester phase.
(5) Crystallization step: crystallizing the fourth ester phase at 40 °C for 20 h, and performing suction filtration to obtain a sucralose crude product and 1.3 L of a fifth ester phase respectively (after 4 times of balance); washing the fifth ethyl ester phase with water for 6 times (the water consumption is 0.55 L each time), wherein the ester phase after washing is a sixth ester phase (the content of sucralose is 0.06 g/L), and carrying out concentration and drying on the sixth ester phase to obtain recovered ethyl acetate and sugar residues respectively; the aqueous phases were combined as a fifth aqueous phase which was used in step (2) instead of water to mix with the first ethyl ester phase concentrate.

In step (6), the ratio of the dry weight of the sucralose crude product to the volume of ethyl acetate is 1 kg: 1.5 L, and the sugar degree of the pulped target product aqueous solution is 30%.

In step (7), the concentration is carried out under the conditions of 60 °C and - 0.1 MPa (gauge pressure), the sugar degree of the pulped and decolored target product concentrated solution is 50%, and the first crystallization time is 16 h.

In step (8), the dissolving temperature is 50 °C, the sugar degree of the first crystallized target product aqueous solution is 55%, and the second crystallization time is 12 h.

In step (9), the dissolving temperature is 40 °C, the sugar degree of the aqueous solution of the second crystallized target product is 20%, the mass of the activated carbon is 0.5% of the dry weight of the sucralose crude product, the decoloring time is 10 min, the concentration is carried out under the conditions of 70 °C and 0.09 MPa (gauge pressure), the sugar degree of the second crystallized decolored target product concentrated solution is 40%, the third crystallization is carried out for 12 h under the condition of 30 °C, and the drying is carried out for 8h under the condition of 50 °C.

In step (10), the purity and yield data of the sucralose refined product obtained by recycling 17 times are shown in table 4.

**Table 4. Purity and yield of sucralose refined product**

| recycling times | mass of refined product/g | purity/% | yield/% | average yield/% |
|---|---|---|---|---|
| 0 | 39.46 | 99.7 | 25.67 | 70.22 |
| 1 | 88.52 | 99.9 | 66.55 | |
| 2 | 124.07 | 99.7 | 85.31 | |
| 3 | 142.39 | 99.7 | 103.34 | |
| 4 | 156.76 | 99.6 | 111.29 | 113.79 |
| 5 | 161.35 | 99.8 | 114.78 | |
| 6 | 162.66 | 99.8 | 115.32 | |
| 7 | 161.26 | 99.6 | 115.89 | |
| 8 | 155.71 | 99.7 | 111.2 | |
| 9 | 163.64 | 99.8 | 113.17 | |
| 10 | 159.94 | 99.9 | 112.26 | |
| 11 | 163.34 | 99.6 | 113.87 | |
| 12 | 163.35 | 99.7 | 115.09 | |
| 13 | 160.88 | 99.8 | 115.03 | |
| 14 | 160.69 | 99.9 | 114.67 | |
| 15 | 156.50 | 99.6 | 113.83 | |
| 16 | 161.38 | 99.7 | 112.46 | |
| 17 | 163.56 | 99.8 | 114.21 | |

| | | | | |
|---|---|---|---|---|
| Note: the theoretical yield is 119.08%. | | | | |

### Example 3

The sucralose refined product was prepared according to the method of example 1, which differs from example 1 in that:
(1) Extraction step: adding 0.85 L of ethyl acetate into 3 L of first aqueous solution, stirring and extracting for 10min at 50 °C, separating phases to obtain an organic phase and a aqueous phase, repeating the extraction step for 6 times on the aqueous phase, combining ester phases after the extraction is finished to obtain a first ethyl ester phase, and the aqueous phase obtained by the last extraction is the first aqueous phase (the content of sucralose-6-acetate is 0.06 g/L, and the first aqueous phase is subjected to high-salt wastewater treatment), wherein the main components of the first solution are shown in Table 5.

**Table 5. Composition of the first solution**

| Components | Content |
|---|---|
| Sucralose-6-acetate | 60.1 g/L |
| Sucralose diester | 4.8 g/L |
| Tetrachlorosucrose-6-acetate | 4.7 g/L |
| NH₄Cl | 80.3 g/L |
| Other organic impurities | 30.4 g/L |
| Water | 82.7 % |

(2) Concentration and drying step: the first ethyl ester phase was concentrated and dried under vacuum at 80 °C, -0.08MPa (abbreviated as concentrated to dryness), and water (1800 mL) was added to the resulting first ethyl ester phase concentrate, and concentrated to 1.8L under the above conditions to obtain an aqueous solution of sucralose-6-acetate (the content of ethyl acetate is 0.41 g/L), which was then transferred to a three-necked flask.
(3) Alkaline hydrolysis step: cooling the obtained sucralose-6-acetate aqueous solution to 3 °C, then dropwise adding a 32 wt% sodium hydroxide aqueous solution), uniformly mixing, carrying out alkaline hydrolysis for 6 h at 3 °C, keeping the pH value of the system to be 12.5 in the alkaline hydrolysis process, heating to 45 °C after the alkaline hydrolysis is finished, then dropwise adding 30 wt% diluted hydrochloric acid to adjust the pH value to be 7, and filtering to obtain a second aqueous phase (2 L, the content of sucralose-6-acetate is 0.05g/L) and filter residues (solid waste treatment).
(4) Separation step: adding 4.1 L of ethyl acetate into the second aqueous phase, stirring and extracting for 10 min at 50 °C, repeating the extraction operation on the obtained aqueous phase, extracting for 6 times totally, combining ester phases obtained by the first to the second extraction to obtain a second ethyl ester phase (8.5 L), and sequentially using the ester phases obtained by the third to the sixth ethyl acetate extraction for the first to the fourth ethyl acetate extraction of the second aqueous phase in the preparation process of the next batch of sucralose crude products. Adding 1.7 L of water into the second ethyl ester phase for washing for 6 times, wherein the aqueous phase obtained by the first washing is a fourth aqueous phase, and the fourth aqueous phase is used in step (2) instead of water to mix with the first ethyl ester phase concentrate; and sequentially using the aqueous phase obtained by the second to the sixth water washing for the first to the fifth water washing of the second ethyl ester phase in the preparation process of the next batch of sucralose crude product, wherein the ester phase obtained after the water washing is the third ethyl ester phase. Concentrating and drying the third ester phase at 80 °C and -0.09 MPa (gauge pressure), adding ethyl acetate (add 3 times, and 0.8 L is added each time) in the concentrating and drying process to dehydrate so as to ensure that the moisture is completely removed, and adding ethyl acetate into the obtained third ester phase concentrate to adjust the sugar degree Bx to be 41% to obtain a fourth ester phase.
(5) Crystallization step: crystallizing the fourth ester phase at 40 °C for 18 h, and performing suction filtration to obtain a sucralose crude product and 1.1 L of a fifth ester phase respectively (after 4 times of balance); washing the fifth ethyl ester phase for 6 times (the water consumption is 0.55 L each time), wherein the ester phase after washing is a sixth ester phase (the content of sucralose is 0.07 g/L), and carrying out concentration and drying on the sixth ester phase to obtain recovered ethyl acetate and sugar residues respectively; the aqueous phases were combined as a fifth aqueous phase which was used in step (2) instead of water to mix with the first ethyl ester phase concentrate.

In step (6), the ratio of the dry weight of the sucralose crude product to the volume of ethyl acetate is 1 kg: 1.2 L, and the sugar degree of the pulped target product aqueous solution is 25%.

In step (7), concentration is carried out under the conditions of 70 °C and -0.09 MPa (gauge pressure), the sugar degree of the pulped and decolored target product concentrated solution is 65%, and the first crystallization time is 8 h.

In step (8), the dissolving temperature is 40 °C, the sugar degree of the first crystallized target product aqueous solution is 40%, and the second crystallization time is 16 h.

In step (9), the dissolving temperature is 60 °C, the sugar degree of the aqueous solution of the second crystallized target product is 30%, the mass of the activated carbon is 0.7% of the dry weight of the sucralose crude product, the decoloring time is 8 min, the concentration is carried out under the conditions of 80 °C and 0.08 MPa (gauge pressure), the sugar degree of the concentrated solution of the second crystallization decoloring target product is 55%, the third crystallization is carried out for 8 h at the temperature of 40 °C, and the drying is carried out for 10 h at the temperature of 45 °C.

In step (10), the purity and yield data of the sucralose refined product obtained by recycling 17 times are shown in table 6.

**Table 6. Purity and yield of sucralose quality**

| recycling times | mass of refined product/g | purity/% | yield/% | average yield/% |
|---|---|---|---|---|
| 0 | 49.64 | 99.8 | 30.38 | 70.77 |
| 1 | 108.23 | 99.7 | 66.17 | |
| 2 | 135.98 | 99.8 | 83.22 | |
| 3 | 169.12 | 99.6 | 103.29 | |
| 4 | 185.38 | 99.6 | 113.22 | 113.2 |
| 5 | 186.91 | 99.7 | 114.27 | |
| 6 | 188.53 | 99.8 | 115.38 | |
| 7 | 184.82 | 99.9 | 113.22 | |
| 8 | 181.71 | 99.7 | 111.09 | |
| 9 | 183.33 | 99.6 | 111.97 | |
| 10 | 186.05 | 99.8 | 113.86 | |
| 11 | 184.25 | 99.8 | 112.76 | |
| 12 | 184.91 | 99.7 | 113.05 | |
| 13 | 183.19 | 99.7 | 112.00 | |
| 14 | 184.28 | 99.8 | 112.78 | |
| 15 | 186.98 | 99.6 | 114.20 | |
| 16 | 184.45 | 99.8 | 112.88 | |
| 17 | 185.99 | 99.9 | 113.94 | |

| | | | | |
|---|---|---|---|---|
| Note: the theoretical yield is 114.79%. | | | | |

The recycling of the mother liquor is one of the important means for improving the yield of the target product and reducing the production cost. The key of the successful recycle is how to separate the impurities enriched in the repeated and cyclic application process from the target product, and the stable operation of the process can be ensured only by continuously separating the impurities from the target product and discharging the impurities out of the process system. Therefore, at which step, in which way the impurities are separated and discharged is the focus of the process. According to the invention, by means of subjecting the sucralose crude product to ethyl acetate pulping treatment, two different stages of activated carbon impurity removal and third crystallization, so that impurities in the crude product and impurities generated by oxidation in the process of recycling crystallized mother liquor can be effectively removed and discharged out of the system.

And also, the research of the invention finds that if all mother liquor generated in the purification process of the sucralose crude product is recycled, impurities can be enriched, so that qualified sucralose refined product can not be obtained, and the phenomenon of crystallization can not occur after the mother liquor is reused for many times. According to different characteristics (such as solvent and impurity content) of mother liquor generated in the preparation steps, part of the mother liquor (pulped mother liquor and primary crystallized mother liquor) is used for preparing the sucralose crude product, and part of the mother liquor (secondary crystallized mother liquor and tertiary crystallized mother liquor) is reused for purifying the sucralose crude product, so that the mother liquor is recycled, the phenomenon that the sucralose cannot be crystallized is not found after the mother liquor is reused for many times, the yield of the sucralose is obviously improved, and the loss of the sucralose in the purification process is reduced.

In conclusion, the preparation method of sucralose refined product can be effectively combined with the method for preparing sucralose crude product by using hydrolysis system, so that mother liquor formed in the preparation process of sucralose refined product can be recycled, the problem of mother liquor resource waste is solved, the amount of waste liquor is reduced, sucralose is recovered to the maximum extent, the yield of sucralose is significantly increased, food-grade sucralose refined products in different forms can be prepared, the production cost is obviously reduced, and the method has a high industrial prospect.

The above description of the embodiments is only intended to facilitate the understanding of the method of the invention and its core idea. It should be noted that, for those skilled in the art, without departing from the principle of the present invention, it is possible to make various improvements and modifications to the present invention, and those improvements and modifications also fall within the scope of the claims of the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the principles and novel features disclosed herein.

## Claims

1. A preparation method of a sucralose refined product **characterized by** comprising the following steps:
(1) Mixing sucralose crude product with ethyl acetate, pulping, and then carrying out solid-liquid separation to obtain a pulped mother liquor and a pulped target product respectively; dissolving the pulped target product in water, and decoloring the obtained aqueous solution of the pulped target product by using activated carbon to obtain a pulped and decolored target product solution;
(2) Concentrating the pulped and decolored target product solution, and performing first crystallization on the obtained pulped and decolored target product concentrated solution to obtain a first crystallized target product and a primary crystallized mother liquor respectively;
(3) Dissolving the first crystallized target product in water, and carrying out second crystallization on the obtained first crystallized target product aqueous solution to obtain a second crystallized target product and a secondary crystallized mother liquor respectively; the secondary crystallized mother liquor is recycled to step (1) for dissolving the pulped target product;
(4) Dissolving the second crystallized target product in water, and performing solid-liquid separation on the obtained second crystallized target product aqueous solution after activated carbon decolorization to obtain a second crystallized decolorized target product aqueous solution and recovered activated carbon respectively; concentrating the second crystallized decolorized target product aqueous solution, and carrying out third crystallization on the obtained second crystallized decolorized target product concentrated solution to obtain a third crystallized target product and a tertiary crystallized mother liquor respectively; drying the tertiary crystallized target product to obtain a sucralose refined product; recycling the tertiary crystallized mother liquor to step (3) for dissolving the first crystallized target product, and recycling the recovered activated carbon to step (1) for decoloring the pulped target product aqueous solution.

2. The preparation method according to claim 1, wherein in step (1), the sucralose crude product comprises the following components in percentage by mass: 85-95% of sucralose, 0.5-1 % of sucralose-6-acetate, 1-3% of ethyl acetate and 1-13.5% of caramel impurities.

3. The preparation method according to claim 1 or 2, wherein in step (1), the ratio of the mass of the sucralose crude product to the volume of the ethyl acetate is 1 kg: 1-1.5 L.

4. The preparation method according to claim 1, wherein in step (1), the dissolving temperature is 30-50 °C;
The sugar degree of the pulped target product aqueous solution is 20-40%.

5. The preparation method according to claim 1, wherein in step (2), the sugar degree of the pulped and decolored target product concentrated solution is 40-65%.

6. The preparation method according to claim 1 or 5, wherein in step (2), the temperature of the first crystallization is room temperature, and the time is 8-16 h.

7. The preparation method according to claim 1, wherein in step (3), the dissolving temperature is 40-60 °C;
The sugar degree of the first crystallized target product aqueous solution is 40-65%.

8. The preparation method according to claim 1 or 7, wherein in step (3), the temperature of the second crystallization is room temperature, and the time is 8-16 h.

9. The preparation method according to claim 1, wherein in step (4), the dissolving temperature is 40-60 °C;
The sugar degree of the second crystallized decolorized target product aqueous solution is 20-30%.

10. The preparation method according to claim 1, wherein in step (4), the sugar degree of the second crystallized decolorized target product concentrated solution is 40-65%.

11. The preparation method according to claim 1 or 10, wherein in step (4), the temperature of the third crystallization is 30-50 °C, and the time is 4-12 h.

12. The preparation method according to claim 1, wherein the preparation method of the sucralose crude product comprises the following steps:
(a) Extracting the raw material liquid by ethyl acetate to obtain a first ethyl ester phase and a first aqueous phase respectively; concentrating the first ethyl ester phase to obtain a first ethyl ester phase concentrate; the raw material liquid is an aqueous solution containing sucralose-6-acetate, sucralose diester and sucralose tetrachloride-6-acetate;
(b) Mixing and dissolving the first ethyl ester phase concentrate and water, and concentrating to obtain a sucralose-6-acetate aqueous solution;
(c) Mixing the sucralose-6-acetate aqueous solution with an alkali metal hydroxide for carrying out an alkaline hydrolysis reaction, neutralizing the obtained reaction solution, and filtering to obtain a second aqueous phase;
(d) Performing ethyl acetate extraction on the second aqueous phase to obtain a second ethyl ester phase and a third aqueous phase respectively;
(e) Washing the second ethyl ester phase with water to obtain a fourth aqueous phase and a third diethyl ester phase respectively; said fourth aqueous phase is recycled to step (b) for dissolving said first ethyl ester phase concentrate;
(f) Mixing the third ethyl ester phase with ethyl acetate, and carrying out azeotropic dehydration to obtain a third ethyl ester phase concentrate; mixing the third ethyl ester phase concentrate with ethyl acetate to obtain a fourth ethyl ester phase;
(g) Crystallizing the fourth ester phase to obtain a sucralose crude product and a fifth ester phase respectively;
(h) Washing the fifth ethyl ester phase with water to obtain a fifth aqueous phase and a sixth ethyl ester phase respectively; the fifth aqueous phase is recycled to step (b) for dissolving the first ethyl ester phase concentrate.

13. The preparation method according to claim 1 or 12, wherein the pulped mother liquor and the primary crystallized mother liquor are recycled for preparing sucralose crude product.

14. The preparation method according to claim 13, wherein during the recycling, the pulped mother liquor is used for dissolving the first ethyl ester phase concentrate, and after primary crystallized mother liquor and the third ethyl ester phase are mixed, azeotropic dehydration is carried out.

15. The preparation method according to claim 12, wherein the sucralose content in the third, fourth, and fifth aqueous phases is independently < 0.5 g/L.

16. The preparation method according to claim 12, wherein in step (a), the temperature of ethyl acetate extraction is 40-60 °C, and the number of extraction times is 5-8; the volume ratio of the raw material liquid to ethyl acetate for single ethyl acetate extraction is 1: 0.2-0.5.

17. The preparation method according to claim 12, wherein in step (b), the content of ethyl acetate in the sucralose-6-acetate aqueous solution is < 0.5 g/L.

18. The preparation method according to claim 12, wherein in step (c), the **pH** value of the alkaline hydrolysis reaction is 11-13, the temperature is 0-10 °C, and the time is 3-6 h.

19. The preparation method according to claim 12, wherein in step (c), the temperature of the neutralization and the filtration is 50-70 °C independently.

20. The preparation method according to claim 12, wherein in step (d), the number of times of ethyl acetate extraction is 4-7; the volume ratio of the second aqueous phase to the ethyl acetate used for single-time extraction is 1: 1-3;
The Ethyl ester phases obtained by the first to the second ethyl acetate extraction are combined as a second ethyl ester phase;
The ethyl acetate phases obtained by the third to the seventh ethyl acetate extraction are used for the ethyl acetate extraction of the second aqueous phase in the next batch of sucralose crude product preparation process.

21. The preparation method according to claim 12, wherein in step (e), the number of times of the water washing is 3-6; the volume ratio of the second ethyl ester phase to the water used for single-time water washing is 1: 0.1-0.3;
The aqueous phase obtained by the first water washing is taken as a fourth aqueous phase;
The aqueous phases obtained by the second to the sixth water washing are used for the water washing of the second ethyl ester phase in the next batch of sucralose crude product preparation process.

22. The preparation method according to claim 12, wherein in step (f), the water content of the third ethyl ester phase concentrate is < 0.5 wt%.

23. The preparation method according to claim 12, wherein in step (f), the sugar degree of the fourth ethyl ester phase is 40-60%.

24. The preparation method according to claim 12 or 23, wherein in step (g), the crystallization temperature is 40-60 °C, and the time is 12-30 h.
